# EUROPÄISCHE PATENTSCHRIFT

(11) **EP 2 262 451 B1**
(45) Veröffentlichungstag und Bekanntmachung des Hinweises auf die Patenterteilung: **24.05.2017**
(21) Anmeldenummer: 09716516.1
(22) Anmeldetag: 02.03.2009
(51) Int. Cl.: A61F 2/88, A61F 2/95, A61M 25/01, A61F 2/24, A61F 2/91, A61F 2/915, A61M 25/06

(54) **STENT, WELCHER VOM EXPANDIERTEN ZUSTAND KONTROLLIERT ERNEUT IN DURCHMESSER VERRINGERBAR IST**
STENT, WHICH CAN BE DECREASED IN DIAMETER AGAIN IN A CONTROLLED MANNER FROM THE EXPANDED STATE
STENT QUI EST APTE À ÊTRE RÉDUIT EN DIAMÈTRE DE FAÇON RÉPÉTÉE CONTRÔLÉE À PARTIR DE L'ÉTAT EXPANSÉ

(30) Priorität: 02.03.2008 DE 102008012113; 10.03.2008 DE 102008013381; 12.03.2008 DE 102008013948
(43) Veröffentlichungstag der Anmeldung: 22.12.2010
(73) Patentinhaber: Venus MedTech (HangZhou), Inc., Hangzhou, Zhejiang (CN)
(72) Erfinder: GÖTZ, Wolfgang, 93051 Regensburg (DE); LIM, Hou Sen, 455234 Singapore (SG)
(74) Vertreter: Bobbert & Partner Patentanwälte PartmbB
(86) Internationale Anmeldenummer: PCT/EP2009/001464
(87) Internationale Veröffentlichungsnummer: WO 2009/109348

(56) Entgegenhaltungen:
- EP-A- 0 732 087
- WO-A-97/21402
- DE-A1- 19 936 207
- FR-A- 2 688 688
- US-A1- 2006 155 366

## Beschreibung

Die vorliegende Erfindung betrifft einen Katheter gemäß dem Oberbegriff des Anspruchs 1. Sie betrifft ferner ein Implantat mit den Merkmalen des Anspruchs 10. Ferner betrifft sie ein Set mit den Merkmalen des Anspruchs 13.

Die Erfindung ist in den Ansprüchen definiert. Alle anderen Ausführungsbeispiele sind lediglich illustrativ. Stents oder Implantate allgemein werden in der Medizin zum Aufhalten von Gefäßen (im Speziellen: Blutgefäßen) oder Röhren (im Speziellen Trachea, Oesophagus, Magen, Darm, Urethra, Urether) benutzt. Sie werden gefaltet oder gecrimpt mittels eines Katheters zur Implantationsstelle vorgeschoben. Die Entfaltung gefalteter Stents erfolgt dort nach Entfernen einer äußeren Hülle (sleeve), die über dem gefalteten Stent angeordnet ist, durch Rückstellkräfte des Stents, oder mittels eines innen liegenden Ballons, der bei seinem Aufblasen den ihn umgebenden Stent aufdehnt. Stents sind gewöhnlich aus biokompatiblem Stahl. Einmal entfaltet oder expandiert ist es schwierig bis unmöglich, den Stent wieder auf einen verringerten Durchmesser zurückzuführen. Letzteres ist regelmäßig dann von Bedeutung, wenn der Stent falsch im Zielorgan (Gefäß oder anderes Organ, siehe oben) platziert entfaltet oder expandiert wurde, denn ohne die Möglichkeit einer Verringerung seines Durchmessers lässt sich ein am verkehrter Ort expandierter oder entfalteter Stent i.a.R. nicht mehr an den ursprünglich beabsichtigten Ort umimplantieren.

Aufgabe der vorliegenden Erfindung ist es, einen Katheter zum Rückführen eines Implantats, insbesondere eines Stents, nach dessen Entfalten und/ oder Expandieren am

Implantationsort auf einen wieder verringerten Durchmesser anzugeben. Ebenso soll ein hierzu geeignetes Implantat, insbesondere ein Stent, angegeben werden. Ferner soll ein Set vorgestellt werden.

Die erfindungsgemäße Aufgabe wird gelöst durch die Merkmalskombination des Anspruchs 1.

Somit wird erfindungsgemäß ein Katheter zum lösbaren Aufnehmen eines expandierbaren und/ oder entfaltbaren Implantats, insbesondere eines Stents, vorgeschlagen, wobei der Katheter wenigstens eine Einrichtung zum Steuern des Expandierens und/ oder des Entfaltens des Implantats von einem ersten Durchmesser auf einen zweiten Durchmesser und/ oder des Rückführens des Implantats vom zweiten Durchmesser auf den ersten Durchmesser aufweist oder vorbereitet ist zur Aufnahme einer solchen Einrichtung.

Unter einem Expandieren oder Entfalten wird erfindungsgemäß ein Vergrößern des Durchmessers des Implantats verstanden. Dabei kann der nicht expandierte oder der nicht entfaltete Durchmesser (auch als erster Durchmesser bezeichnet werden kann, wobei auch ein anderer Durchmesser, welcher kleiner als der unten stehen beschriebene zweite Durchmesser ist, als erster Durchmesser im Sinne der vorliegenden Erfindung verstanden werden kann) ein solcher des Implantats unmittelbar vor einem Einbringen in den Patientenkörper sein. Beim Rückführen des Durchmessers von einem zweiten Durchmesser (welcher größer als der erste Durchmesser ist) auf einen beliebigen verringerten Durchmesser (den ersten Durchmesser), wird der Durchmesser verkleinert. Das Rückführen kann mit einem erneuten Falten (vollständig oder teilweise) oder einem umgekehrten Expandiervorgang einhergehen. Beim Rückführen muss das Implantat erfindungsgemäß jedoch nicht zwingend in Formen gebracht werden, welcher es auch beim Entfalten oder Expandieren inne gehabt oder durchlaufen hat.

Sollte sich ein Durchmesser des Implantats nicht bestimmen lassen, so wird unter Expandieren oder Entfalten eine Zunahme in einer Richtung oder Dimension des Implantats verstanden, welche eine Verlängerung eines Umfangs des Implantats in einer Ebene senkrecht zur weiter unten erläuterten Längsrichtung des Implantats bewirkt.

Unter einem Aufnehmen eines Implantats durch den Katheter wird erfindungsgemäß jede funktionelle Verbindung zwischen Implantat und Katheter verstanden. Eine Übertragung von Kräften kann dabei erfolgen, muss jedoch nicht. Die Verbindung kann kraftschlüssig oder formschlüssig oder weder kraftschlüssig noch formschlüssig vorgesehen sein. "Steuern" umfasst erfindungsgemäß auch ein Regeln. Dabei kann auf einen Spannungswert, Druckwert oder dergleichen geregelt werden.

Unter einem "lösbaren Aufnehmen" wird erfindungsgemäß eine trennbare Vereinigung zwischen Katheter und Implantat verstanden. Ein Beispiel einer trennbaren Vereinigung ist das Crimpen eines Stents auf einen Katheter zum Vorschieben des Stents an die Implantationsstelle.

Der erfindungsgemäße Katheter erlaubt vorteilhaft eine kontrollierte Entfaltung und wieder Faltung (womit auch ein Expandieren und ein Rückführen auf einen verringerten Durchmesser gemeint sein kann) des Implantats, z. B. bei seiner Anordnung in einem Inneren des Implantats. Somit ist es vorteilhaft möglich, das Implantat nach dessen Expansion oder Entfaltung erneut auf einen geringeren Durchmesser zu bringen und dadurch umzuimplantieren, also an der Implantationsstelle zu verschieben. Sollte sich bei der Implantation herausstellen, dass ein Implantat falscher Größe oder Bauweise gewählt wurde, so kann vorteilhaft ein Austausch auch noch nach Expansion/Entfaltung erfolgen.Ein solcher Katheter wird z. B. in Dokumenten WO 97/21402 oder FR 2 688 688 beschrieben. Dabei kann der Katheter mittels der Einrichtung zum Steuer mit dem Implantat in Verbindung steht. Die Entfaltung und Faltung des Implantats kann ohne Zuhilfenahme einer äußeren Hülle (sleeve) erfolgen.

Der Katheter kann einen Kunststoff oder ein Polymer oder ein Copolymer aufweisen oder in Zwei- oder Mehrkomponententechnik gefertigt sein. Der Katheter kann auch ein Metall (Stahl oder Legierung) aufweisen. Der Katheter kann steif sein, er kann jedoch auch biegsam ausgestaltet sein, um ihn an bestimmte Gegebenheiten anpassen zu können. Dabei kann der Katheter mit der Hand biegbar sein oder mittels eines Mechanismus, welche z.B. in den Katheter integriert sein kann, zu einer Biegung steuerbar sein. Der Katheter kann passiv biegbar sein, z. B. allein durch sein Vorschieben entlang des Gefäßes oder Körperlumens.

Der Katheter kann losgelöst aller weiteren Merkmale, d.h. ohne diese weiteren Merkmale ebenfalls in Kombination aufweisen zu müssen, einen mechanisch verstärkten Abschnitt, insbesondere in einem Spitzenbereich des Katheters und insbesondere in einem Abschnitt, welcher auch wenigstens eine der Durchlasseinrichtungen aufweist.

Der Katheter kann einen kreisrunden oder ovalen oder rechteckigen Querschnitt aufweisen. Ebenso kann der Katheter einen nicht-kreisrunden, einen nicht-ovalen oder einen nicht-rechteckigen Querschnitt aufweisen. Zudem kann der Querschnitt des Katheters über den gesamten Katheter hinweg unverändert sein. Er kann jedoch auch zwei oder drei oder mehr verschiedene Querschnitte entlang seiner Längsachse und insbesondere im Bereich zur Aufnahme des Implantats aufweisen.

Die durch den Katheter kontrollierte Entfaltung und Faltung des Implantats kann außerhalb und innerhalb eines Patientenkörpers stattfinden.

Der Katheter kann ausgestaltet sein, wie bspw. in der US 2007/0100427 A1 von Perouse oder der US 2005/0075731 A1 von Artof et al. beschrieben ist.

Der Katheter kann einlumig sein, er kann kein Lumen aufweisen und er kann mehrlumig sein. Ist er mehrlumig, so kann er zwei- oder drei- oder mehrlumig mit gleich großen oder unterschiedlich großen Lumina im Querschnitt sein.

Vorteilhafte Weiterbildungen der vorliegenden Erfindung sind jeweils Gegenstand der Unteransprüche.

So wird in einer bevorzugten Ausführungsform der vorliegenden Erfindung ein Katheter mit einer Mehrzahl von Lumen in Längsrichtung oder Kanälen (im Folgenden auch einfach kurz: Kanal oder Kanäle) zum Führen von Zügeln vorgeschlagen. Die Lumen oder Kanäle können zum Organisieren und/oder Ordnen der Zügel dienen. Sie können in vorteilhafter Weise sicher stellen, dass der Arzt stets feststellen kann, welchen der ggf. gleich aussehenden Zügel er gerade in der Hand hält oder betätigen möchte. Er muss sich hierzu nur daran orientieren, aus welchem Kanal der in Frage stehende Zügel austritt oder in welchen er eintritt.

Insbesondere können die Kanäle zum Verhindern eines Verwirrens, Verhedderns, Verknotens usw. der Zügel mit- oder untereinander dienen.

In den Kanälen können ferner jeweils Zügel zusammengefasst sein, welche bei ihrer Betätigung einem gleichen oder gemeinsamen Zweck dienen. So können durch einen Kanal Zügel geführt werden, welche allesamt zum Bewirken eines bestimmten Verhaltens des Implantats oder des Katheters bedient werden müssen. Durch einen anderen Kanal können Zügel geführt werden, welche zum Bewirken eines anderen Verhaltens des Implantats oder des Katheters bedient werden müssen. Dies erleichtert dem Arzt die Bedienung des Implantats oder des Katheters mittels der Zügel ersichtlich. Es können so auch die Zügel die zum Implantat laufen von den Zügeln, die von dem Implantat zurücklaufen getrennt werden.

Das Vorsehen der Zügel in einer Mehrzahl von Kanälen und ihre Führung in diesen kann ferner eine gegenseitige Beeinträchtigung verhindern oder ein Risiko hierzu verringern. So kann bei entsprechender Anordnung der Zügel in getrennten Kanälen beispielsweise sicher gestellt werden, dass durch das Ziehen an einem Zügel, welcher in einem ersten Kanal verläuft, nicht aufgrund von Reibung oder einer anderen Wechselwirkung des Zügels mit einem weiteren Zügel dieser weitere Zügel unbeabsichtigter Weise mitbetätigt wird.

Das Vorsehen von einer Mehrzahl von Kanälen zum getrennten Führen von Zügeln kann ferner vorteilhaft eine Trennung von Zügeln und anderen Einrichtungen wie beispielsweise einem Führungsdraht ermöglichen. Die Funktion der Zügel ist somit nicht durch weitere Einrichtungen und Funktionen des Katheters beeinträchtigt, aber - und dies ist nicht minder vorteilhaft - auch umgekehrt; d.h. es kann vorteilhaft auch verhindert werden, dass die weitere Einrichtung wie ein Führungsdraht durch die Anwesenheit oder Betätigung der Zügel des Katheters beeinträchtigt wird.

Das Vorsehen von einer Mehrzahl von Kanälen zum getrennten Führen von Zügeln kann somit vorteilhaft die Präzision beim Betätigen der Zügel und damit das Verwenden des Katheters oder des Implantats erhöhen.Ein Katheter wird mit wenigstens einer Durchlasseinrichtung zum Durchlassen eines oder mehrerer Zügel vorgeschlagen. Die Zügel dienen zum Beeinflussen des Expandierens und/ oder des Entfaltens des Implantats mittels Veränderung einer Spannung, welcher der Zügel auf das Implantat ausübt. Dabei wird erfindungsgemäß unter einem Durchlassen auch ein Durchführen verstanden. Eine Durchlasseinrichtung kann eine Durchlassöffnung, eine Öse, ein Umlenkabschnitt oder dergleichen sein.

Unter einem Zügel kann erfindungsgemäß ein Polymer, ein Metall oder ein biologisches Fadenmaterial aufweisen oder hieraus bestehen. Der oder die Zügel können resorbierbar sein.

Unter Spannung, welche der Zügel auf das Implantat ausübt, wird erfindungsgemäß auch ein Zug oder allgemein jede Wirkung des Zügels auf das Implantat verstanden.

Wenn in der vorliegenden Anmeldung von einem Zügel die Rede ist, so können auch mehr als ein Zügel gemeint sein, beispielsweise zwei, drei, vier, fünf oder mehr.

Die Zügel können funktionell auch getrennt vom Katheter getrennt vorliegen.

Der Katheter kann eine innere Führungseinrichtung für den wenigstens einen Zügel aufweisen.

Die Zügel können den Katheter in einer bevorzugten Ausführungsform durch eine oder mehrere Durchlasseinrichtungen verlassen (speziell, wenn diese als Durchlassöffnungen ausgestaltet sind) auf einer Seite und/ oder an einem Ende des Katheters. Diese Durchlasseinrichtungen können sich auf einer oder auf mehreren Ebenen quer zur Längsachse des Katheters befinden.

Der Katheter kann eine Vorrichtung zum Durchtrennen der Zügel aufweisen.

Das Implantat kann ein Stent, insbesondere ein Klappen tragender Stent, insbesondere ein Herzklappen tragender Stent sein.

Die erfindungsgemäße Aufgabe wird auch gelöst durch die Merkmalskombination des Anspruchs 10. Erfindungsgemäß wird dabei ein Implantat, insbesondere ein Stent, vorgeschlagen, das zum lösbaren Aufnehmen durch den Katheter nach Anspruch 1 geeignet ist. Die hiermit erzielbaren Vorteile entsprechen zumindest jenen des erfindungsgemäßen Katheters. Daher wird zur Vermeidung von Wiederholungen an dieser Stelle auf deren Diskussion verwiesen.Das Implantat weist wenigstens eine Führungseinrichtung auf, welche vorbereitet und geeignet ist zum Führen von wenigstens einem Zügel, mittels welchem wenigstens ein Abschnitt des Implantats durch Verändern von Zug oder Spannung auf dem Zügel vom ersten Durchmesser auf den zweiten Durchmesser expandierbar und/ oder entfaltbar und/ oder vom zweiten Durchmesser auf den ersten Durchmesser rückführbar ist

Das Implantat kann selbstexpandierend sein, bspw. aus oder mit einem Memory Material, im speziellen Nitinol, oder Materialien, welche Nitinol aufweisen. Das Implantat kann jedoch auch teils selbstexpandierend, teils mittels einer Einrichtung zum Expandieren expandierbar sein. Das Implantat kann ausschließlich nicht-selbstexpandierend sein. Das Implantat kann faltbar sein, das Implantat kann nicht-faltbar sein.

Das Implantat kann ein biokompatibles Material, insbesondere einen biokompatiblen Edelstahl, aufweisen. Das Material kann bioabsorbierbar sein.

Das Implantat kann mit einer oder ohne eine Einrichtung zum Umfassen oder Sandwichen von Teilen von nativen Klappenabschnitten (insbesondere Herzklappensegel) ausgestaltet sein. Insbesondere kann das Implantat mit Abschnitten oder ohne Abschnitte ausgestaltet sein, welche sich aufgrund von Temperatur und Memory-Effekt erheben oder senken.

Die eine oder mehrere Führungseinrichtungen des Implantats können in Form von Führungslöchern, Führungsringen, Ösen, Haken oder ganz allgemein Führungsstrukturen ausgestaltet sein. Sie geben dem Zügel eine Führung, welche im Sinne eines Lenkens des Zügels in einer Richtung verstanden werden kann. Unter Führung kann erfindungsgemäß auch verstanden werden, dass der Zügel eine Stabilisierung in seinem Verlauf erfährt. Dabei kann der Zügel durch die Führungseinrichtung von einem Inneren des Implantats oder Stents im speziellen vom Katheter zur Implantatstruktur (insbesondere zur Außenseite des Implantats) geführt werden. Die Führungseinrichtung des Implantats kann symmetrisch (insbesondere rund, oval oder eckig) oder asymmetrisch sein. Die Führungseinrichtung kann sich auf einer Ebene, auf mehreren Ebenen oder auf einer spiralförmigen Ebene des Implantates befinden. Mehrere Führungseinrichtungen können gleich ausgestaltet sein, oder wenigstens in zwei verschiedenen Ausgestaltungen vorliegen.

Das Implantat oder der Stent kann eine zirkuläre Führungseinrichtung aufweisen. Diese kann in Form eines Kanals, welche bezogen auf das Implantat nach außen, nach oben oder nach unten offen oder geschlossen ist, ausgestaltet sein. Die Führungseinrichtung, kann geschlossen oder offen sein und eine symmetrische oder asymmetrische Form besitzen. Die Führungseinrichtung kann als eine Gitterstruktur, eine Mäanderstruktur, eine Sinuswellenstruktur, insbesondere mit 18 Wellenspitzen entlang eines Umfangs, oder dergleichen ausgestaltet sein. Die Führungseinrichtung kann eine Struktur ohne ein Gitter und/ oder ohne ein Mäander und/ oder ohne eine Sinuswellenstruktur aufweisen. Sie kann jedoch auch eine Sinuswellenstruktur mit einer anderen Anzahl an Wellenspitzen als 18 aufweisen.

Beim Implantat kann es sich um einen Klappen tragenden Stent aus Stahl handeln, wie in den Patenten US 5,411,552, US 5,840,081 und US 6,168,614 B1 von Andersen et al. beschrieben ist. Es kann aber auch um einen Klappen tragenden selbst-expandierenden Stent gemäß der Offenbarung der US 7,018,406 B2 von Seguin et al. oder der US 2005/0075731 A1 von Artof et al. handeln.

Die Zügel werden entlang eines Inneren des Katheters geführt und den Katheter durch die Durchlasseinrichtung verlassen. Anschließend werden die Zügel durch die Führungseinheiten am Implantat geführt. Die Zügel werden entlang eines Umfangs oder Teilen eines Umfanges in der Führungseinheit entlang der Implantat-Zirkumferenz geführt. Die Zügel werden anschließend wieder durch eine Durchlasseinrichtung von außen nach innen in den Katheter zurückgeführt. Die Zügel können den Katheter an seinem chirurgennahen Ende verlassen. Die Zügel können den Katheter an seinem chirurgenfernen Ende verlassen. Die Wirkung und insbesondere der Zug der Zügel und somit die kontrollierte Entfaltung und erneute Faltung oder Verringerung im Durchmesser des Stents kann mittels einer Steuereinheit gesteuert werden.

Ein Zügel kann über eine Durchlasseinrichtung am Katheter diesen verlassen und mittels der selben Durchlasseinrichtung oder mittels einer anderen Durchlasseinrichtung auf der gleichen oder auf einer anderen Ebene wieder zurück in den Katheter geführt werden. Die Zügel können mit beiden Enden den Katheter verlassen.

Die Zügel können mit einem Ende den Katheter verlassen und mit dem anderen Ende mit dem Katheter verbunden sein.

Nach Durchtrennen oder einem Ausklinken oder anderem anderen Auslösen der Zügel können diese vom Implantat oder vom Stent zurückgezogen und entfernt werden.

Die Zügel können durch eine Vorrichtung im Katheter, eine Vorrichtung innerhalb oder außerhalb des Katheters (insbesondere durch ein Messer, eine Schere, durch elektrische Spannung, durch Hitze) durchtrennt werden. Eine geeignete Vorrichtung kann am Katheter vorliegen.

Bei geringer oder ohne Spannung der Zügel ist der Stent entfaltet oder expandiert. Bei Spannung der Zügel wird der Stent im Durchmesser verringert oder erneut teilweise oder vollständig gefaltet.

Die erfindungsgemäße Aufgabe wird auch gelöst durch die Merkmalskombination des Anspruchs 14. Erfindungsgemäß wird dabei ein Set vorgeschlagen, welches zumindest ein erfindungsgemäßes Implantat, insbesondere ein Stent, und wenigstens einen wie oben definierten erfindungsgemäßen Katheter aufweist.

Die hiermit erzielbaren Vorteile entsprechen zumindest jenen des erfindungsgemäßen Katheters. Daher wird zur Vermeidung von Wiederholungen an dieser Stelle auf deren Diskussion verwiesen.

In einer bevorzugten Ausführungsform des Sets ist der Katheter im Zentrum oder im Wesentlichen im Zentrum eines Querschnitts oder eines jeden Querschnitts des Implantats angeordnet.

Diese Anordnung im Zentrum bzw. in der Mitte kann sich auf einen Gebrauchszustand beziehen. Sie kann sich auf einen nicht entfalteten oder nicht expandierten Zustand beziehen. Sie kann sich jedoch auch auf einen vollständig expandierten oder entfalteten Zustand beziehen.

Ferner kann der Gebrauchszustand jener während eines Positionierens des Implantats sein - insbesondere bei dessen Rotieren um einen Drehmittelpunkt hiervon - zum exakten Herstellen der gewünschten Lagebeziehung zwischen Implantat und dem anantomischen Ort, an welchem das Implantat im Körper implantiert werden oder zum Liegen kommen soll.

Das Anordnen des Katheters im Zentrum des Implantats kann eine Reihe von Vorteilen mit sich bringen, zu welchen ein gleichmäßiges Expandieren des Implantats zählt. Ferner kann das Implantat bei zentriertem Katheter besser gesteuert und positioniert werden. Folgendes Beispiel mag dies verdeutlichen: Im Falle einer Herzklappenprothese als Implantat kann es erforderlich sein, während des Einbringens der auf dem Katheter gehaltenen Prothese (dem Implantat) diese in Bezug auf die Kommissuren der Aortenwurzel derart auszurichten, dass deren Ausrichtung und die Ausrichtung der an der Prothese vorhandenen Klappensegel aufeinander abgestimmt sind. Zu diesem Zweck wird der Katheter vom Arzt um seine Längsachse gedreht, und diese Drehung bringt bei zentral angeordnetem Katheter auch die Herzklappenprothese in eine gewünschte Stellung in Bezug auf ihre Ausrichtung in ihrer Rotationsrichtung. Ist der Katheter dabei nicht im Zentrum der rotationssymmetrisch aufgebauten Herzklappenprothese angeordnet, so erfährt diese eine Auslenkung in einer radialen oder lateralen Richtung. Diese Auslenkung erschwert nicht nur das Erzielen der gewünschten Ausrichtung in Drehrichtung, sie übt auch eine unerwünschte Kraft und Beanspruchung von umgebenden Strukturen wie der Aortenwurzel, einer bereits eingesetzten Aufnahmeeinrichtung zum Aufnehmen und Verankern der Prothese und dergleichen aus. Das Zentrieren des Katheters in der Mitte des Implantats kann dem vorteilhaft entgegenwirken. Die relative Beziehung zwischen Katheter und Implantat kann unverändert bleiben. Ein Auftreten unerwünschter Auslenkungen und Kräfte kann vorteilhaft vermieden werden.

Das Zentrieren des Katheters in der Mitte des Implantats bzw. sein Anordnen im Zentrum des Implantats kann vorteilhaft genutzt werden beim Überprüfen der Klappenfunktion bei noch mit dem Implantat verbundenem Katheter. Die Klappensegel können sich ohne Behinderung durch den im Zentrum angeordneten Katheter für eine Funktionsprüfung entfalten und schließen, was nicht möglich wäre, wäre der Katheter bezogen auf einen Querschnitt des Herzklappenprothese randständig angeordnet. Eine andere Position des Katheters als die zentrale Position könnte zu einem ungleichmäßigen Öffnen und Schließen der Klappensegel führen und eine Funktionsprüfung erschweren, in ihrem Ergebnis verzerren oder unmöglich machen. Eine derartige Funktionsprüfung bei noch mit der Herzklappe verbundenem Katheter ist aber von großer Bedeutung und großem Nutzen, da eine Revision oder ein Umpositionieren der Klappe bei festgestellter, unbefriedigender Lage möglich sein soll.

Eine zentrale Lage des Katheters bezogen auf den Querschnitt des Implantats kann auch in einem zusammengefalteten oder nicht expandierten Zustand des Implantats von Vorteil sein, da diese Lage eine Vereinfachung des Faltens, Crimpens oder dergleichen des Implantats erlauben kann. Dabei kann der Platzbedarf vorteilhaft verringert werden, eine Beschädigung des Implantats oder Abschnitten hiervon (wie Klappensegel in einer Ausführungsform) kann vorteilhaft verhindert werden, usw.

Eine zentrale Lage des Katheters bezogen auf den Querschnitt des Implantats kann ferner vorteilhaft ein Steuern der Lage des Implantats derart ermöglichen, dass dieses mit seinem Umfang die Struktur, in welche es eingesetzt werden soll, gleichmäßig berührt. Spannungs- oder Kraftspitzen, welche das aufnehmende Gewebe beschädigen oder den Vorgang des Einsetzens des Implantats erschweren können, lassen sich auf diese Weise vorteilhaft vermeiden. Die Gefahr von Beschädigungen des Implantats (Beispiel Klappensegel) oder Verletzungen von Gewebe kann hierbei vorteilhaft verringert werden. Die Erfindung wird anhand der beigefügten Zeichnung exemplarisch erläutert. Dabei werden gleiche oder ähnliche Strukturen über die Figuren hinweg mit denselben Bezugszeichen bezeichnet. In der Zeichnung gilt:
- Fig. 1: zeigt schematisch vereinfacht einen Katheter mit Führungseinheiten;
- Fig. 2: zeigt die Anordnung der Fig. 1;
- Fig. 3: zeigt die Anordnung der Fig. 1;
- Fig. 4: zeigt die Anordnung der Fig. 1;
- Fig. 5: zeigt einen exemplarischen Verlauf von drei Zügeln um je 1/3 des Stentumfangs;
- Fig. 6: zeigt Verlauf der Zügel um den vollständigen Stentumfang;
- Fig. 7: zeigt den Katheter der Fig. 5;
- Fig. 8: zeigt den Katheter der Fig. 6 mit dem Stent der Fig. 7;
- Fig. 9: zeigt einen expandierbaren und in seinem Durchmesser durch eine Einrichtung wieder verringerbaren Stent;
- Fig. 10: zeigt den Stent der Fig. 9;
- Fig. 11: zeigt angespannte Zügel und einen verringerten Durchmesser des Stents;
- Fig. 12: zeigt einen Stent in der Darstellung der Fig. 9;
- Fig. 13: zeigt einen Stent in der Darstellung der Fig. 10;
- Fig. 14: zeigt eine weitere Ausführungsform, bei welcher die Zügel in einer spiralförmigen Form um den Stent geführt werden;
- Fig. 15: zeigt den Zügel an beiden Enden angezogen, wobei sich der Durchmesser des spiralförmig geführten Zügels verringert hat;
- Fig. 16: zeigt den Zustand des Zügels der Fig. 15 um einen Stent herum; und
- Fig. 17: zeigt einen Katheter in einem Querschnitt mit einer Mehrzahl von Lumen bzw. Lunima.

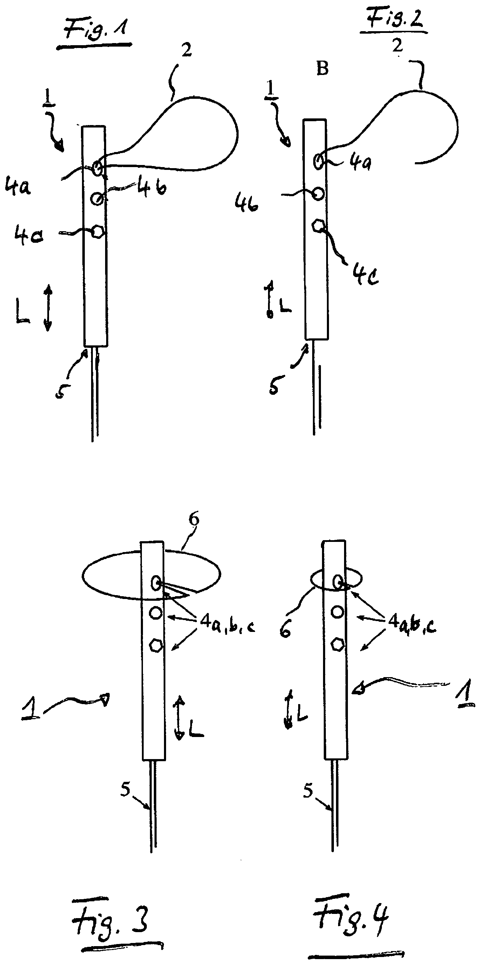

Fig. 1 zeigt einen Katheter 1 mit einem Zügel 2, welcher in Richtung einer Längsachse L des Katheters 1 in diesen eintritt und durch Durchlasseinrichtungen 4a, 4b und 4c, welche auch einer Führung des Zügels 2, welcher beispielsweise als Faden ausgestaltet sein kann, dienen können, auf einer oder mehreren Ebenen oder Abschnitten des Katheters 1 austritt. Die Durchlasseinrichtungen 4 können symmetrisch oder asymmetrisch sein. Sie können rund (4b), oval (4a), eckig (4e) oder in jeder anderen geeigneten Form ausgestaltet sein. Der Zügel 2, welcher im Beispiel der Fig. 1 durch eine Längsöffnung 5 in den Katheter 1 eintritt, kann nach Austritt aus der Durchlasseinrichtung 4a eine geschlossene Schlinge bilden, deren anderes Ende bei derselben Durchlasseinrichtung 4a, welche hier als Einlasseinrichtung und zugleich als Auslasseinrichtung dient, wieder in den Katheter 1 eintritt und diesen beispielsweise ebenfalls durch die Längsöffnung 5 des Katheters 1 verlässt.

Zwischen der Spitze (oben in der Fig. 1) und der Längsöffnung 5 liegt ein Hohlraum 9 des Katheters 1 durch welchen der oder die Zügel 2 verlaufen können. Der Hohlraum 9 kann sich zumindest von der Längsöffnung 5 bis zur (in Fig. 1) obersten Durchlassöffnung 4a erstrecken.

Der Katheter 1 kann losgelöst aller weiteren Merkmale, d. h. ohne diese weiteren Merkmale ebenfalls in Kombination aufweisen zu müssen, einen mechanisch verstärkten Abschnitt, insbesondere in einem Spitzenbereich des Katheters und insbesondere in einem Abschnitt, welcher auch wenigstens eine der Durchlasseinrichtungen aufweist.

Fig. 2 zeigt die Anordnung der Fig. 1, wobei der Zügel 2 durchtrennt wurde und nun von einem nicht dargestellten Stent und aus dem Katheter 1 zurückgezogen werden kann.

Fig. 3 zeigt wiederum die Anordnung der Fig. 1, wobei der Zügel 2 in einem Bogen 6 um den (nicht dargestellten) Stent geführt und kehrt durch dieselbe Durchlasseinrichtung 4a in den Katheter 1 zurückgeführt. Der Zügel 2 hat keine oder nur eine geringe Spannung, bei welcher der Stent entfaltet sein kann.

Fig. 4 zeigt erneut die Anordnung der Fig. 1 und insbesondere jene der Fig. 3, wobei der Zügel 2 in Fig. 4 angespannt ist. Der Durchmesser des (nicht dargestellten) Stents hat sich aufgrund der Einwirkung des Bogens 6 erneut verringert. Der Stent ist im Falle eines faltbaren Stents wieder teilweise oder ganz zusammengefaltet.

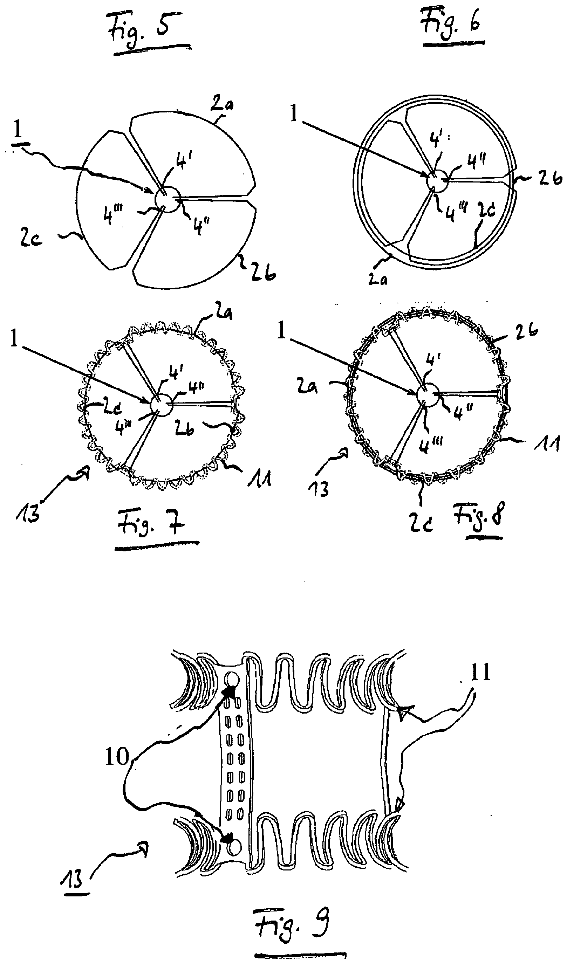

Fig. 5 zeigt einen exemplarischen Verlauf von drei Zügeln 2a, 2b und 2c um je 1/3 des Stentumfangs. Jeder dieser Zügel verlässt den Katheter durch eine Durchlasseinrichtung 4', 4" oder 4'" und kehrt durch eine hiervon verschiedene Durchlasseinrichtung zurück.

Fig. 6 zeigt Verlauf der Zügel 2a, 2b und 2c um den vollständigen Umfang. Jeder dieser Zügel tritt aus dem Katheter 1 aus einer Durchlasseinrichtung 4', 4" oder 4"' aus und tritt durch dieselbe erneut in den Katheter 1 ein.

Fig. 7 zeigt den Katheter 1 der Fig. 5. Die Zügel 2a, 2b und 2c verlassen den Katheter 1 und werden um 1/3 des Umfangs in einer Führungseinrichtung 11 des Stents 13 (hier exemplarisch ein C-förmiger, halboffener Kanal) geführt.

Fig. 8 zeigt den Katheter 11 der Fig. 6 mit dem Stent 13 der Fig. 7. Die Zügel 2a, 2b und 2c verlassen den Katheter 1 und werden jeweils durch die Führungseinrichtung 11 entlang der gesamten Zirkumferenz bzw. des gesamten Umfangs des Stents 13 wieder zur gleichen Durchlasseinrichtung 11 am Katheter (1) zurück geführt.

Fig. 9 zeigt einen expandierbaren und in seinem Durchmesser (in einer Ebene senkrecht zu einer Längsachse des Stents, welche im wesentlichen einer laminaren Strömungsrichtung des Bluts eines ungekrümmten Gefäßes, in welches der Stent eingesetzt ist, entspricht; die Längsrichtung entspricht auch der Richtung der größten Räumlichen Erstreckung des Stents vor seiner Entfaltung außerhalb des Körpers des Patienten sowie nach seiner Entfaltung in einem vergleichsweise geraden Gefäßabschnitt) durch eine nicht gezeigte Einrichtung wieder verringerbaren Stent 13.

Dieser Stent 13 weist zwei zirkuläre Führungseinrichtungen 11 in Form jeweils eines nach außen halboffenen Kanals - dieser kann auch zum Stent hin offen sein - und zwei Durchlasseinrichtungen 10 in Form von runden Durchlässen auf (dabei können die Durchlasseinrichtungen 10 auch in jeder Ausführungsform und losgelöst von allen weiteren Merkmalen nicht-rund ausgestaltet sein und allein, zu zweit, zu dritt oder zu mehreren vorliegen). Der Stent 13 kann ferner auch eine andere Anzahl von Führungseinrichtungen 11 als zwei, beispielsweise eine, drei, vier oder mehr, aufweisen.

Die Führungseinrichtungen 11 können dabei zirkulär angeordnet sein, sie können aber auch nicht-zirkulär vorgesehen sein. Die Führungseinrichtungen 11 können einstückig mit dem Stent gefertigt sein, oder auch getrennt davon. Ebenso können die Durchlasseinrichtungen 10 einstückig mit dem Stent gefertigt sein, oder ebenfalls getrennt davon. Die Führungseinrichtungen 11 können in Wellenform ausgestaltet sein, sie können aber auch in jeder anderen Form, insbesondere nicht-wellenförmig gefertigt sein.

Die Durchlasseinrichtungen 10 sind in Abschnitten von Streben 12 angeordnet. Sie können an gegenüberliegenden Enden der Streben 12 angeordnet sein, aber auch in jedem anderen Abschnitt, beispielsweise in einem mittleren Bereich und nicht an den Enden der Streben. Sie können ferner auch an einer anderen Stelle des Stents 13 als in oder an den Streben 12 vorgesehen sein. Durch die Durchlasseinrichtungen 10 hindurch können nicht dargestellte Zügel von einem Inneren des Stents 13 nach außen und wieder zurückgeführt werden.

Der Stent 13 als Beispiel eines Implantats kann eine beliebige Anzahl von Streben 12 aufweisen, welche alle in gleicher Bauart oder in wenigstens zwei unterschiedlich ausgestalteten Bauarten vorgesehen sind. Die Streben 12 können dabei unter jeweils gleichem Abstand voneinander beabstandet sein. Sie können jedoch auch wenigstens zwei voneinander verschiedene Abstände zueinander aufweisen. Die Streben 12 können zwar die Durchlassöffnungen 10 aufweisen, letztere können jedoch auch getrennt von den Streben 12 vorgesehen sein. Ebenso können die Streben 12 Öffnungen aufweisen, sie können jedoch auch ohne Durchgangsöffnungen ausgestaltet sein. Der Stent 13 kann mit Streben ausgestaltet sein, welche keine Durchlassöffnungen 10 aufweisen. Er kann ferner wenigstens eine Strebe mit Durchlassöffnungen und wenigstens eine Strebe ohne Durchlassöffnungen aufweisen. Der Stent kann wenigstens eine Strebe aufweisen, welche keinerlei Durchgangsöffnungen hat. Der Stent kann wenigstens eine Strebe aufweisen, welche zur Längsrichtung des Stents geneigt im oder am Stent angeordnet ist. Die Streben 12 können dabei an ihren beiden Enden nach außen gebogen verlaufen. Sie können jedoch ungeachtet anderer Merkmale nicht oder zumindest nicht an beiden Enden nach außen gebogen verlaufen. Die Streben 12 können mit ihren beiden Enden mit wellenförmigen Strukturen des Stents verbunden sein. Sie können jedoch ungeachtet anderer Merkmale nicht oder zumindest nicht an beiden Enden mit wellenförmigen Strukturen verbunden sein.

Der Stent 13 kann losgelöst von allen weiteren Merkmalen aus flachem Material gefertigt - z. B. mit dem Laser geschnitten - sein, welche z. B. nach dem Ausgestalten eines Musters im flachen Material zu einem Rohr umgeformt wird (ggf. unter Verbinden wie Verschweißen von Längsseiten der ehemals flachen Materialbahn). Er kann aber auch direkt aus einem rohrförmigen Material gefertigt werden.

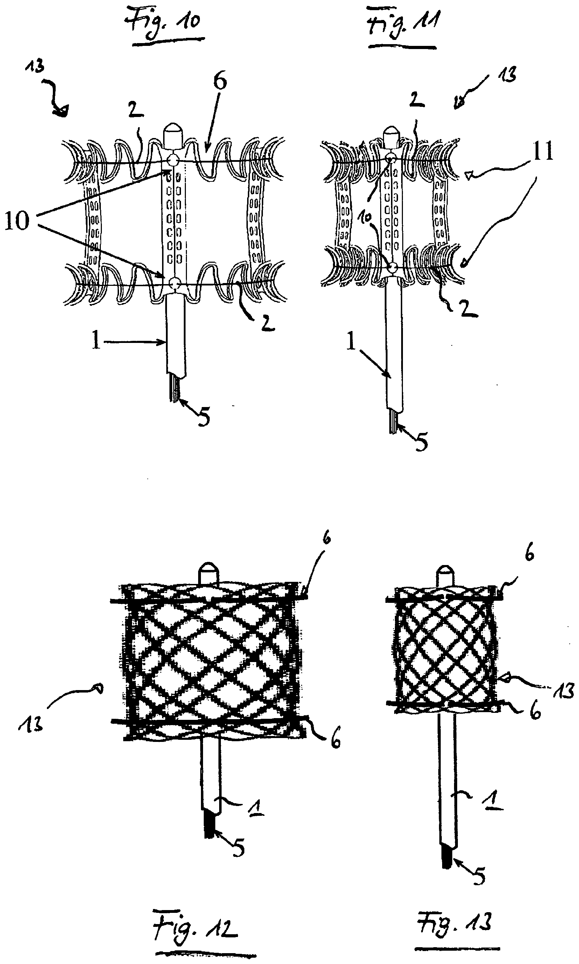

Fig. 10 zeigt den Stent 13 der Fig. 9. Zwei Zügel 2 wurden um den Stent 13 geführt und kehren durch jeweils dieselbe Führungseinrichtung 10 zum Katheter 1 zurück. Die Zügel 2 üben auf den Stent 13 keine oder nur eine geringe Spannung aus und der Stent 13 ist entfaltet.

In Fig. 11 sind die Zügel 2 angespannt. Der Durchmesser des Stents 13 hat sich verringert. Der Stent 13 ist wieder vermehrt zusammengefaltet. Die Zügel 2 werden in der Führungseinrichtung 11 des Stents 13 geführt

Die Fig. 12 und 13 zeigen einen Stent 13 in Darstellungen, welche den Darstellungen der Fig. 9 und Fig. 10 entsprechen. Dabei zeigt Fig. 9 den Stent 13 in expandiertem Zustand, Fig. 10 zeigt ihn mit wieder verringertem Durchmesser. Der in den Fig. 9 und 10 gezeigte Stent kann dabei jedem bekannten Stent (mit oder ohne Klappen) entsprechen. Insbesondere kann der Stent 13 jedem bekannten entfaltbaren Stent entsprechen.

Fig. 12 zeigt schematisch vereinfacht zudem eine Steuereinheit 14 zum kontrollierten Entfalten oder Expandieren und erneuten Falten oder Verringern im Durchmesser des Stents 13.

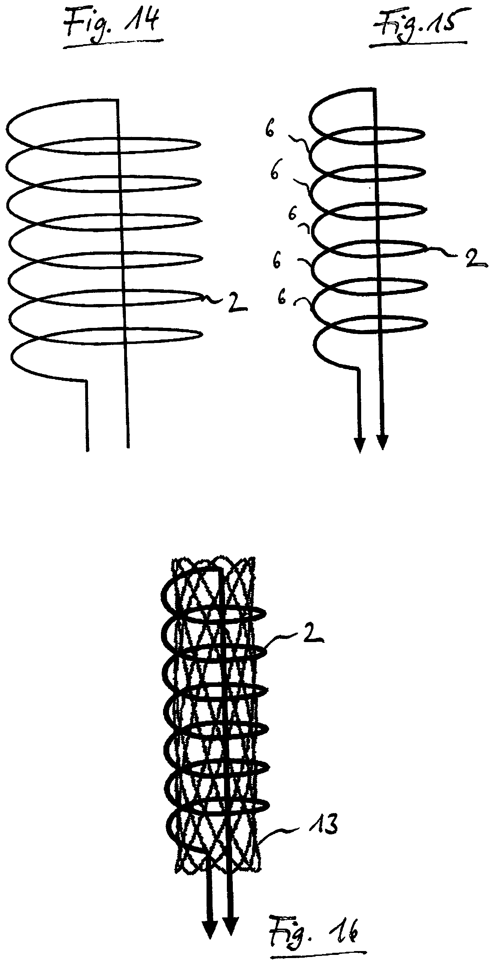

Fig. 14 zeigt, dass die Zügel 2 auch in einer spiralförmigen Form um den Stent 13 geführt werden können. Hierbei ist nur ein Zügel 2 gezeigt, der im Inneren des Stents 13 zu dessen vorderen Abschnitt geführt wird. Anschließend wird der Zügel 2 außen über den Stent 13 geführt und tritt erneut zurück in den nicht gezeigten Katheter 1. Der Zügel 2 ist ohne oder nur mit geringer Spannung und der (nicht dargestellte Stent) ist entfaltet.

In Fig. 15 ist der Zügel 2 an beiden Enden in Pfeilrichtung angezogen, und der Durchmesser des Bogens 6 des spiralförmig geführten Zügels 2 hat sich verringert.

Fig. 16 zeigen den Zustand des Zügels 2 der Fig. 15. Der Zügel 2 (welche hier auch als Faden bezeichnet werden) wurde angespannt, und der Stent 13 wurde mittels des spiralförmig geführten Zügels 2 zusammengefaltet.

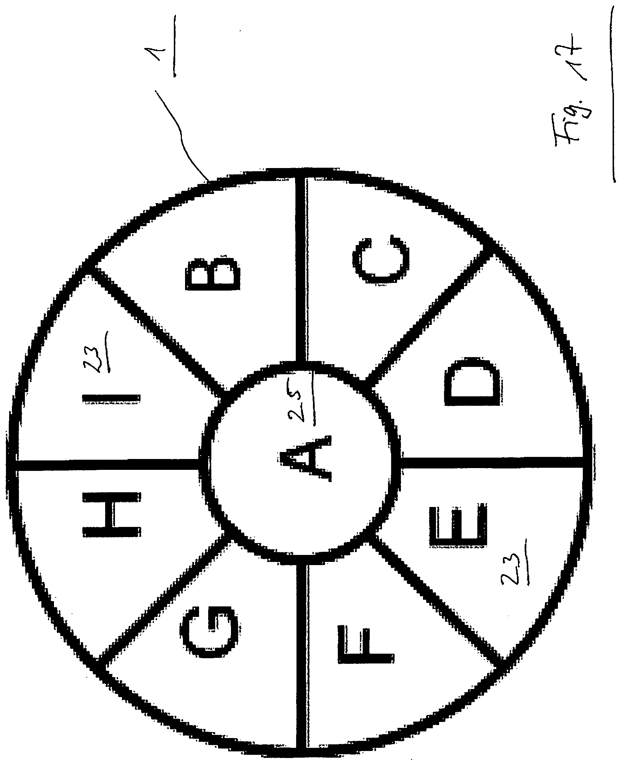

Fig. 17 zeigt einen Katheter 1 mit einer Mehrzahl von Lumen oder Kanälen 23 zum Hindurchführen von in Fig. 17 nicht dargestellten Zügeln in einem Querschnitt. Diese Kanäle 23 für die Zügel sind mit den Buchstaben B, C, D, E, F, G und H bezeichnet.

Die Lumen können geeignet und vorbereitet sein zum Hindurchführen von Zügeln zum Gebrauch des Katheters.

Der Katheter 1 weist in seiner Mitte bzw. in seinem Zentrum ein weiteres Lumen 25 auf, welche mit dem Buchstaben A bezeichnet ist und in der gezeigten Ausführungsform vorgesehen ist, eine in Fig. 17 ebenfalls nicht gezeigte Einrichtung wie einen Führungsdraht aufzunehmen.

Das weitere Lumen 25, welches in Fig. 17 in der Mitte des Querschnitts des Katheters 1 gezeigt ist, ist nicht auf diese Anordnung festgelegt. Lumen oder Kanäle (ein oder mehrere) können auch am Rand des Querschnitts liegen, zwei von ihnen können aus Handhabungsgründen einander gegenüberliegen usw. Der Katheter kann zudem mehr als nur ein Lumen 25 für jeweils eine oder mehrere weitere Einrichtung(en) aufweisen.

Bei der weiteren Einrichtung 25 kann es sich um eine Einrichtung handeln, welche kein Zügel ist und/oder welcher keine Funktion beim Verändern des Durchmessers des Implantats übernimmt.

Bei der weiteren Einrichtung 25 kann es sich um eine Einrichtung zum Durchtrennen der Zügel handeln.

## Patentansprüche

1. Katheter (1) zum lösbaren Aufnehmen eines expandierbaren und/oder entfaltbaren Implantats, insbesondere Stents (13), wobei der Katheter (1) wenigstens einen Zügel (2a, 2b, 2c) sowie wenigstens eine Einrichtung zum Steuern des Expandierens und/oder des Entfaltens des Implantats von einem ersten Durchmesser auf einen zweiten Durchmesser und dessen Rückführens vom zweiten Durchmesser auf den ersten Durchmesser mittels des wenigstens einen Zügels (2a, 2b, 2c) aufweist, **dadurch gekennzeichnet, dass** die Einrichtung zum Steuern mehrere seitliche Durchlasseinrichtungen (4a, 4b, 4c, 4', 4", 4"', 5) zum Durchlassen eines oder mehrerer Zügel (2a, 2b, 2c) aufweist, welche in verschiedenen Ebenen quer zur Längsachse des Katheters (1) angeordnet sind, wobei das Expandieren und/oder das Entfalten des Implantats mittels einer Veränderung einer Spannung des Zügels (2a, 2b, 2c) steuerbar ist, welche der Zügel (2a, 2b, 2c) auf das Implantat ausübt, und dadurch, dass einer der Zügel (2a, 2b, 2c) geeignet ist, durch eine der Durchlasseinrichtungen (4a, 4b, 4c, 4', 4", 4"', 5) ein Inneres des Katheters (1) zu verlassen, entlang eines Umfangs oder Teilen eines Umfanges entlang einer Implantat-Zirkumferenz geführt zu werden und wieder durch eine der Durchlasseinrichtungen (4a, 4b, 4c, 4', 4", 4"', 5) von außen nach innen in den Katheter (1) durch dieselbe oder durch eine andere Durchlasseinrichtung (4a, 4b, 4c, 4', 4", 4"', 5) zurückgeführt zu werden.

2. Katheter (1) nach Anspruch 1, welcher wenigstens eine Durchlasseinrichtung, insbesondere eine Durchlassöffnung (5), zum Eintreten des Zügels (2a, 2b, 2c) in einen offenen oder geschlossenen langgestreckten Hohlraum (9) des Katheters (1) aufweist.

3. Katheter (1) nach Anspruch 2, wobei der langgestreckte Hohlraum (9) am proximalen und/oder am distalen Ende des Katheters (1) angeordnet ist.

4. Katheter (1) nach einem der vorangegangenen Ansprüche, welcher wenigstens eine aufblasbare Einrichtung, insbesondere einen aufblasbaren Ballon, zum Expandieren des Implantats aufweist.

5. Katheter (1) nach einem der vorangegangenen Ansprüche, wobei der Zügel (2a, 2b, 2c) um 1/3 des Umfangs des Implantats führbar und in eine andere Durchlasseinrichtung (4a, 4b, 4c, 4', 4", 4"', 5) zurückführbar ist.

6. Katheter (1) nach einem der vorangegangenen Ansprüche, wobei der Zügel (2a, 2b, 2c) um den vollständigen Umfangs des Implantats führbar und in dieselbe Durchlasseinrichtung (4a, 4b, 4c, 4', 4", 4"', 5) zurückführbar ist.

7. Katheter (1) nach einem der vorangegangenen Ansprüche, welcher geeignet und/oder vorbereitet ist für eine Verbindung mit einer Steuereinheit (14) zum Steuern der Einrichtung zum Steuern des Expandierens und/oder des Entfaltens des Implantats von einem ersten Durchmesser auf einen zweiten Durchmesser und/oder dessen Rückführens vom zweiten Durchmesser auf den ersten Durchmesser, insbesondere zum Steuern des auf dem Zügel (2) herrschenden Zugs und/oder der auf dem Zügel (2) herrschenden Spannung.

8. Katheter (1) nach einem der vorangegangenen Ansprüche, welcher eine Mehrzahl von Kanälen (23) oder Lumen aufweist zum Hindurchführen von Zügeln (2).

9. Katheter (1) nach einem der vorangegangenen Ansprüche, welcher wenigstens ein Lumen oder einen Kanal (25) zum Hindurchführen einer weiteren Einrichtung aufweist.

10. Implantat, insbesondere Stent (13), geeignet zum lösbaren Aufnehmen durch einen Katheter nach Anspruch 1, mit wenigstens einer Führungseinrichtung (11), welche vorbereitet und geeignet ist, zum Führen von wenigstens einem Zügel (2), mittels welchem wenigstens ein Abschnitt des Implantats durch Verändern von Zug oder Spannung auf dem Zügel (2) vom ersten Durchmesser auf den zweiten Durchmesser expandierbar und/oder entfaltbar und/oder vom zweiten Durchmesser auf den ersten Durchmesser rückführbar ist.

11. Implantat nach Anspruch 10, welches als Klappen tragender Stent, insbesondere als Herzklappen tragender Stent, ausgestaltet ist.

12. Implantat nach einem der Ansprüche 10 bis 11, welches ein Memory-Shape-Material, insbesondere Nitinol, aufweist.

13. Set, welches wenigstens einen Katheter (1) nach einem der Ansprüche 1 bis 9 und wenigstens ein expandierbares und/oder entfaltbares Implantat, insbesondere einen Stent (13), nach einem der Ansprüche 10 bis 12 aufweist.

14. Set nach Anspruch 13, bei welchem der Katheter im Zentrum eines Querschnitts des Implantats angeordnet ist.

## Claims

1. A catheter (1) for detachably receiving an expandable and/or decollapsible implant, in particular stents (13), wherein the catheter (1) comprises at least one rein (2a, 2b, 2c) as well as at least one device for controlling the expansion and/or the decollapsing of the implant from a first diameter to a second diameter and its return back from the second diameter to the first diameter by means of the at least one rein (2a, 2b, 2c),
**characterized in that** the device for controlling comprises several lateral passage means (4a, 4b, 4c, 4', 4", 4"', 5), for letting pass one or more reins (2a, 2b, 2c), which are arranged in different planes transversely to the longitudinal axis of the catheter (1), wherein the expanding and/or the decollapsing of the implant is controllable by means of a change of a tension of the reins (2a, 2b, 2c), which the reins (2a, 2b, 2c) exert on the implant, and **in that** one of the reins (2a, 2b, 2c) is suitable to leave an interior of the catheter (1) through one of the passage means (4a, 4b, 4c, 4', 4", 4"', 5), to be guided along a circumference or parts of a circumference along an implant circumference and to be guided back again through one of the passage means (4a, 4b, 4c, 4', 4", 4"', 5) from outside to inside into the catheter (1) through the same or another passage
means (4a, 4b, 4c, 4', 4", 4"', 5).

2. The catheter (1) according to claim 1, comprising at least one passage means, in particular a passage opening (5), for inserting the rein (2a, 2b, 2c) in an open or closed elongated void (9) of the catheter (1).

3. The catheter (1) according to claim 2, wherein the elongated void (9) is arranged at the proximal and/or at the distal end of the catheter (1).

4. The catheter (1) according to anyone of the preceding claims comprising at least one inflatable means, in particular an inflatable balloon, for expanding the implant.

5. The catheter (1) according to anyone of the preceding claims, wherein the rein (2a, 2b, 2c) can be guided along 1/3 of the circumference of the implant and can be guided back into another passage
means (4a, 4b, 4c, 4', 4", 4"', 5).

6. The catheter (1) according to anyone of the preceding claims, wherein the rein (2a, 2b, 2c) can be guided along the full circumference of the implant and can be guided back into the same passage
means (4a, 4b, 4c, 4', 4", 4"', 5).

7. The catheter (1) according to anyone of the preceding claims which is suited and/or prepared for a connection with a control device (14) for controlling the means for controlling the expansion and/or the decollapsing of the implant from a first diameter to a second diameter and/or its return back from the second diameter to the first diameter, in particular for controlling the tension and/or the strain applied to the rein (2).

8. The catheter (1) according to anyone of the preceding claims comprising a plurality of channels (23) or lumina for guiding reins (2) through.

9. The catheter (1) according to anyone of the preceding claims comprising at least one lumen or one channel (25) for guiding a further means through.

10. An implant, in particular a stent (13), suitable for the releasable reception through one catheter according to claim 1, comprising at least one guiding means (11) which is prepared and suited for guiding at least one rein (2) by means of which at least one section of the implant is expandable and/or decollapsible from the first diameter to the second diameter and/or is returnable back from the second diameter to the first diameter by changing the tension or strain applied to the rein (2).

11. The implant according to claim 10 which is embodied as a valve supporting stent, in particular as a heart valve supporting stent.

12. The implant according to any one of the claims 10 to 11 comprising a memory shape material, in particular nitinol.

13. A set comprising at least one catheter (1) according to anyone of claims 1 to 9 and at least one expandable and/or decollapsible implant, in particular a stent (13) according to anyone of claims 10 to 12.

14. The set according to claim 13 in which the catheter is arranged in the centre of a cross-section of the implant.

## Revendications

1. Un cathéter (1) prévu pour recevoir un implant extensible et/ou pliable de façon détachable, en particulier un stent (13), où le cathéter (1) présente au moins une bride (2a, 2b, 2c) ainsi qu'au moins un dispositif prévu pour contrôler l'expansion et/ou le pliage de l'implant d'un premier diamètre à un second diamètre ainsi que son retour du second diamètre au premier diamètre au moyen d'au moins une bride (2a, 2b, 2c),
**caractérisé en ce que** le dispositif prévu pour contrôler présente plusieurs moyens de passage (4a, 4b, 4c, 4', 4", 4"', 5) latéraux pour laisser passer une ou plusieurs brides (2a, 2b, 2c), lesquelles sont agencées transversalement à l'axe longitudinal du cathéter (1) dans différents plans, où l'expansion et/ou le pliage de l'implant est contrôlable au moyen d'une modification de la tension de la bride (2a, 2b, 2c) que la bride (2a, 2b, 2c) exerce sur l'implant, et **en ce qu'**une des brides (2a, 2b, 2c) est apte à quitter l'intérieur du cathéter (1) par l'un des moyens de
passage (4a, 4b, 4c, 4', 4", 4"', 5), à être guidée le long d'une périphérie, ou d'une partie de la périphérie, le long de la circonférence de l'implant et à être ramenée vers l'intérieur du cathéter (1) par l'un des moyens de passage (4a, 4b, 4c, 4', 4", 4"', 5), soit par le même, soit par un moyen de passage (4a, 4b, 4c, 4', 4", 4"', 5) différent.

2. Le cathéter (1) selon la première revendication comprenant au moins un moyen de passage, en particulier une ouverture de passage (5), pour introduire la bride (2a, 2b, 2c) dans une cavité (9) allongée ouverte ou fermée du cathéter (1).

3. Le cathéter (1) selon la seconde revendication où la cavité (9) allongée est agencée à l'extrémité distale ou proximale du cathéter (1).

4. Le cathéter (1) selon l'une quelconque des revendications précédentes présentant au moins un moyen gonflable, en particulier un ballon gonflable, prévu pour élargir l'implant.

5. Le cathéter (1) selon l'une quelconque des revendications précédentes où la bride (2a, 2b, 2c) peut être guidée sur un tiers de la périphérie de l'implant et peut être ramenée par un autre moyen de passage (4a, 4b, 4c, 4', 4", 4"', 5).

6. Le cathéter (1) selon l'une quelconque des revendications précédentes où la bride (2a, 2b, 2c) peut être guidée sur la totalité de la périphérie de l'implant et peut être ramenée par le même moyen de passage (4a, 4b, 4c, 4', 4", 4"', 5).

7. Le cathéter (1) selon l'une quelconque des revendications précédentes apte à et/ou préparé pour une connexion avec une unité de contrôle (14) afin de contrôler le dispositif de contrôle d'extension et/ou de pliage de l'implant d'un premier diamètre à un second diamètre et/ou son retour du second diamètre au premier diamètre, en particulier prévu pour contrôler la traction ou la tension exercée sur la bride (2).

8. Le cathéter (1) selon l'une quelconque des revendications précédentes comprenant une pluralité de canaux (23) ou de conduits pour le passage des brides (2).

9. Le cathéter (1) selon l'une quelconque des revendications précédentes comprenant au moins un conduit ou un canal (25) pour le passage d'un dispositif supplémentaire.

10. Un implant, en particulier un stent (13), apte à la réception détachable par un cathéter (1) selon la première revendication avec au moins un dispositif de guidage (11) préparé et apte à guider au moins une bride (2) au moyen de laquelle au moins une section de l'implant est extensible et/ou pliable d'un premier diamètre à un second diamètre et/ou inversement d'un second diamètre à un premier diamètre au moyen de la modification de la traction ou de la tension sur la bride (2).

11. L'implant selon la revendication 10 équipé comme stent porteur de valvules, en particulier comme stent porteur de valvules cardiaques.

12. L'implant selon l'une quelconque des revendications 10 à 11 comprenant un matériau à mémoire de forme, en particulier du nitinol.

13. Un set comprenant au moins un cathéter (1) selon l'une quelconque des revendications 1 à 9 et au moins un implant extensible et/ou pliable, en particulier un stent (13), selon l'une quelconque des revendications 10 à 12.

14. Le set selon la revendication 13 dans lequel le cathéter est agencé au centre de la section transversale de l'implant.
